Europäisches Patentamt

European Patent Office

Office européen des brevets

(11) Veröffentlichungsnummer: **0 258 566 B1**

# (12) EUROPÄISCHE PATENTSCHRIFT

(45) Veröffentlichungstag der Patentschrift: **13.11.91**

(51) Int. Cl.5: **A61M 5/32**, A61M 5/14

(21) Anmeldenummer: **87109534.5**

(22) Anmeldetag: **02.07.87**

(54) **Punktionskanüle.**

(30) Priorität: **22.08.86 DE 8622507 U**

(43) Veröffentlichungstag der Anmeldung:
**09.03.88 Patentblatt 88/10**

(45) Bekanntmachung des Hinweises auf die
Patenterteilung:
**13.11.91 Patentblatt 91/46**

(84) Benannte Vertragsstaaten:
**AT BE CH DE ES FR GB IT LI NL SE**

(56) Entgegenhaltungen:
**DE-U- 8 436 619**
**FR-A- 2 556 224**
**US-A- 3 240 373**
**US-A- 3 774 606**
**US-A- 4 170 993**

(73) Patentinhaber: **B. Braun Melsungen AG**
**Carl-Braun Strasse**
**W-3508 Melsungen(DE)**

(72) Erfinder: **Zahn, Jörn**
**Hermann-Mattern-Strasse 8**
**W-3500 Kassel(DE)**
Erfinder: **Mohr, Georg**
**Wiesenweg 16**
**W-6444 Wildeck-Hönebach(DE)**

(74) Vertreter: **Selting, Günther, Dipl.-Ing. et al**
**Deichmannhaus am Hauptbahnhof**
**W-5000 Köln 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft eine Punktionskanüle, bestehend aus einem Kunststoffkapillar, das an seinem patientenfernen Ende einen hohlen Katheteransatz aufweist und aus einer in das Kunststoffkapillar einschiebbaren Stahlkanüle mit einem Kanülenansatz, der mit dem Katheteransatz zusammensteckbar und in zusammengestecktem Zustand durch eine Verriegelungsvorrichtung sicherbar ist, die mindestens einen äußeren radialen Vorsprung an dem Katheteransatz sowie mindestens einen Einführungsschlitz und eine Ausnehmung zur Aufnahme des Vorsprunges in einem Ringbundteil des Kanülenansatzes aufweist.

Eine solche Punktionskanüle ist bekannt (DE-GM 84 36 619). In diesem Falle ist die Ausnehmung in dem Ringbundteil des Kanülenansatzes als umfangsmäßiger Haken ausgebildet, der gegen den Einführungsschlitz offen ist. Nach der Art eines Bajonettverschlusses wird der radiale Vorsprung an dem Katheteransatz axial in den Einführungsschlitz des Ringbundteiles eingeschoben und durch Drehung des Katheteransatzes relativ zum Kanülenansatz wird der Vorsprung hinter den Haken geschoben, so daß er diesen hintergreift und ein axiales Auseinanderziehen von Katheteransatz und Kanülenansatz verhindert. Eine feste Verbindung zwischen diesen beiden Teilen ist beispielsweise bei der Punktion der Vena Subclavia zum Legen eines Cava-Katheters wichtig, bei der häufig zum Auffinden der Vene Knochenkontakt gesucht wird. Der Anwender schiebt dabei die komplette Punktionskanüle mehrmals vor und zurück und es muß verhindert werden, daß während dieses Vor- und Zurückschiebens die Stahlkanüle zum Teil aus dem Kunststoffkapillar herausrutscht, weil dies bei erneutem Vorschieben zu einem Aufstauchen des Kunststoffkapillars bzw. zu einer Beschädigung der Spitze des Kunststoffkapillars beim Nachschieben der Stahlkanüle führen kann. Solche Deformationen und Beschädigungen des Kunststoffkapillars sind unerwünscht, weil sie die Sicherheit der Subclavia-Punktion beeinträchtigen und den Patienten belasten und gefährden. Die bekannte Verriegelungsvorrichtung läßt sich zwar einfach handhaben, jedoch ist eine ungewünschte Dekonnektion des Kanülenansatzes und des Katheteransatzes bei der Punktion nicht zuverlässig ausgeschlossen, weil bereits eine geringfügige Drehung beider Teile relativ zueinander ein Herausrutschen des Katheteransatz-Vorsprunges aus dem Kanülenansatz-Haken verursacht.

Den Zusammenhalt von zwei Teilen beschreibt US-A-3 240 373 bei einem Sicherheitsverschluß für Flaschen, der aus einem ebenen Schieber mit einer Schnappsicherung besteht. Die Schnappsicherung weist an dem Schieber eine zu seiner Ebene federnd auslenkbare Zunge auf, die an ihrem Ende einen nach oben gerichteten Vorsprung trägt, der in einen Schlitz im Flaschenoberteil eingreift. Durch Herunterdrücken der Zunge mit Hilfe einer Münze oder dgl. (Figur 12) wird der Vorsprung aus dem Schlitz herausgedrückt und der Schieber läßt sich zur Öffnung des Flaschenausgusses bewegen. Bei der Verschiebung des Schiebers zum Verschluß der Flaschenöffnung schnappt der Vorsprung selbsttätig in den Schlitz hinein. Die Verriegelung und Entriegelung der Schnappsicherung dieses Sicherheitsverschlusses einer Flasche läßt sich beliebig oft wiederholen. Eine drehfeste Verbindung zwischen zwei rotationssymmetrischen Teilen wird hierbei allerdings weder angestrebt noch erreicht.

Der Erfindung liegt die Aufgabe zugrunde, die Verriegelungsvorrichtung der bekannten Punktionskanüle so zu verbessern, daß sie während der Punktion den Zusammenhalt von Katheteransatz und Kanülenansatz zuverlässig gewährleistet.

Diese Aufgabe wird erfindungsgemäß dadurch gelöst, daß die Ausnehmung als geschlossene Lochöffnung ausgebildet ist, in die der auf der Außenfläche und an seinen beiden Enden nockenartig abgerundete radiale Vorsprung durch Relativdrehung von Katheteransatz und Kanülenansatz einrastend einführbar ist, und daß der der Lochöffnung zugewandte Seitenrand des Einführungsschlitzes lippenartig abgeflacht ist.

Auf diese Weise wird erreicht, daß der in die geschlossene Lochöffnung eingerastete Vorsprung an allen Seiten von den Rändern der Lochöffnung umgeben ist und nur unter Überwindung eines radialen Druckwiderstandes aus dem Eingriff lösbar ist. Bei der normalen Handhabung der Punktionskanüle während des Punktionsvorganges genügt dieser Druckwiderstand zur Gewährleistung des Zusammenhaltes von Katheteransatz und Kanülenansatz, d.h. ein Auseinanderrutschen von Kunststoffkapillar und Stahlkanüle wird zuverlässig verhindert. Nach Einschieben der Stahlkanüle in das Kunststoffkapillar wird der radiale Vorsprung des Katheteransatzes in den Einführungsschlitz im Ringbund des Kanülenansatzes eingeschoben und sodann wird durch eine Drehbewegung um ca. 70° der Vorsprung gegen die Lochöffnung des Ringbundteils bewegt, bis sie in diese einrastet. Die dem Einführungsschlitz zugewandte Begrenzung der Lochöffnung im Ringbundteil bildet einen Steg, der während des Verbindungsvorganges elastisch um die Höhendifferenz zwischen dem Vorsprung-Außenradius und dem Ringbundteil-Innenradius angehoben wird und beim Einrasten des Vorsprunges in die Lochöffnung zurückfedert, so daß eine sichere formschlüssige Verbindung hergestellt wird. Zur Dekonnektion beider Ansätze wird der Vorsprung aus der Lochöffnung herausgedreht, wobei die radiale Haltekraft des Steges der Lochöffnung überwunden werden muß und Kanülenansatz und Katheteransatz können axial voneinander abgezogen werden, sobald der Vorsprung den Einführungsschlitz des Ringbundteils erreicht hat. Zweckmäßigerweise sind zwei einander diametral gegenüberliegende Vorsprünge an dem Katheteransatz und zwei entsprechend angeordnete Lochöffnungen an dem Ringbundteil des Kanülenansatzes vorgesehen.

Die maximal mögliche Zahl der Konnektionen und Dekonnektionen ist von der Werkstoffelastizität und der geeigneten Formgebung in den Kantenbereichen abhängig. Um Kerbwirkungen zu minimieren und bei ausreichender Haltewirkung glatte Übergänge beim Einrasten bzw. Ausrasten des Vorsprunges zu erzielen, ist erfindungsgemäß vorgesehen, daß der Vorsprung auf der Außenfläche und an seinen beiden Enden nockenartig abgerundet ist und daß der der Lochöffnung zugewandte Seitenrand des Einführungsschlitzes lippenartig abgeflacht ist. Bei Vorhandensein von zwei Lochöffnungen und zwei Einführungsschlitzen sind demgemäß beide Seitenränder des Einführungsschlitzes zugespitzt abgeflacht und als leicht wegdrückbare Lippe gestaltet. Zur Vereinfachung der Dekonnektion kann auch der dem Einführungsschlitz zugewandte Seitenrand der Lochöffnung lippenartig abgeflacht sein.

Erfindungsgemäß soll der Grund des Einführungsschlitzes auf gleicher Umfangslinie mit dem patientenfernen Rand der Lochöffnung liegen. Auch hierdurch wird die Handhabung vereinfacht, weil es genügt, beim axialen Zusammenstecken von Katheteransatz und Kanülenansatz den Vorsprung des Katheteransatzes einfach bis zum Anschlag gegen den Grund des Einführungsschlitzes zu schieben und dann zu drehen.

Der Vorsprung ist in Draufsicht keilförmig gestaltet und die Form der Lochöffnung ist der Form des Vorsprunges im wesentlichen angepaßt, so daß sich ein formschlüssiger Zusammengriff der Rastteile ergibt.

In der Zeichnung ist ein Ausführungsbeispiel der Erfindung schematisch dargestellt.

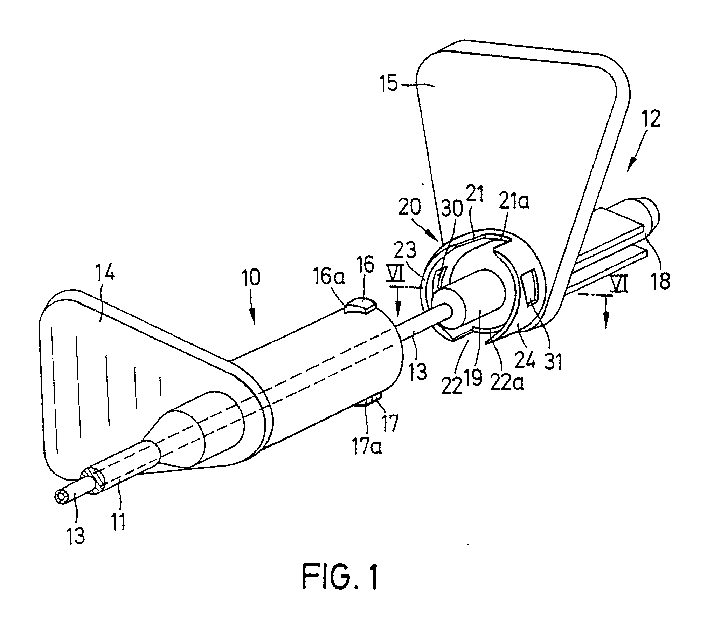

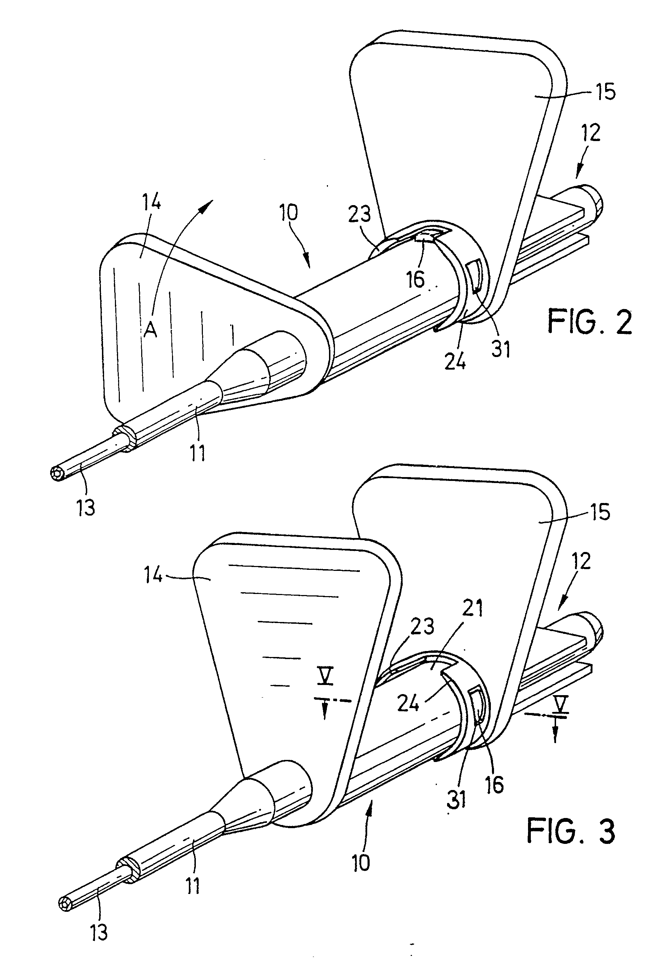

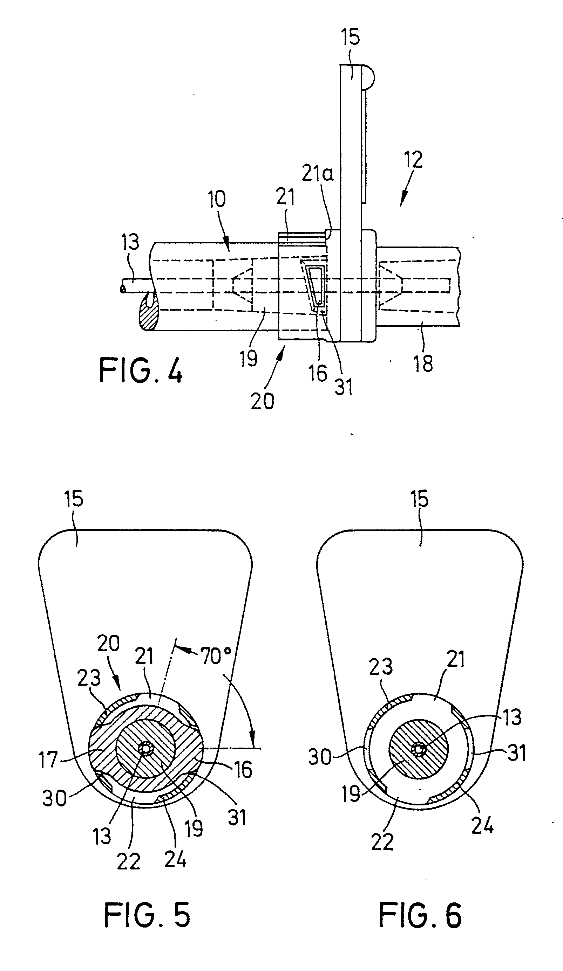

Es zeigen:
Fig. 1, 2 und 3
perspektivische Ansichten verschiedener Stadien der Verbindung eines Kanülenansatzes mit einem Katheteransatz,
Fig. 4
eine schematische Seitenansicht eines Teiles der Punktionskanüle gemäß Figur 3,
Fig. 5
einen Querschnitt durch die Punktionskanüle gemäß Figuren 3 und 4 längs der Linie V-V in Figur 3 und
Fig. 6
einen Schnitt längs der Linie VI-VI in Figur 1.

Die Punktionskanüle besteht im wesentlichen aus einem hohlen Katheteransatz 10 mit einem Kunststoffkapillar 11 und einem Kanülenansatz 12, an dessen patientenseitigem Ende eine spitze angeschärfte Stahlkanüle 13 befestigt ist. Sowohl an dem Katheteransatz 10 als auch an dem Kanülenansatz 12 ist eine zu der Längsachse der Anordnung quergerichtete Griffplatte 14, 15 ausgebildet, die das Anfassen der Punktionskanüle während des Punktionsvorganges sowie die Handhabung der einzelnen Teile erleichtern.

An dem äußeren Rand des Katheteransatzes 10 sind zwei einander diametral gegenüberliegende Vorsprünge 16, 17 ausgebildet, die radial vom Außenumfang des Katheteransatzes 10 abstehen und deren der Spitze des Kunststoffkapillars 11 zugewandte Flanke 16a bzw. 17a in bezug auf die zu dem geraden Rand des Katheteransatzes parallele Flanke schräg verläuft, so daß sich in Draufsicht eine Keilform ergibt. Bei dem in den Figuren veranschaulichten Beispiel weisen die verjüngten Enden der Vorsprünge 16 und 17 - in Richtung der Spitze der Punktionskanüle gesehen - in Gegenuhrzeigersinnrichtung.

Der Kanülenansatz 12 ist auf der hinteren Seite der Griffplatte 15 mit einem axialen Hohlstutzen 18 versehen, in dem die Stahlkanüle 13 offen mündet und der von einem Blutfängerstopfen verschlossen ist, der den Erfolg der Punktion anzeigt. Auf der Vorderseite der Griffplatte 15 ist der Kanülenansatz 12 mit einem hülsenförmigen Ringbundteil 20 ausgestattet, das einen Außenkonus 19 mit Abstand koaxial umgibt. Das Ringbundteil 20 ist dünnwandig und bei gewisser Steifheit rückfedernd elastisch. Das zylindrische Ringbundteil 20 ist durch zwei auf einer durch die Mitte der Griffplatte 15 verlaufenden senkrechten Linie liegende U-förmige Einführungsschlitze 21, 22 in zwei teilkreisförmige Schalen 23, 24 unterteilt, deren umfangsmäßige Länge etwa gleich ist. Die Einführungsschlitze 21, 22 werden von parallelen Seitenrändern begrenzt und ihre Breite ist der Umfangslänge der Vorsprünge 16, 17 angepaßt, so daß diese axial in die Einführungsschlitze 21, 22 einführbar sind, bis sie gegen den Grund 21a bzw. 22a der Einführungsschlitze 21 bzw. 22 anstoßen. In jeder Schale 23, 24 des Ringbundteiles 20 ist eine geschlossene Lochöffnung 30, 31 ausgebildet, deren Kontur im wesentlichen der Keilform der Vorsprünge 16, 17 des Katheteransatzes angepaßt ist. Die parallelen Ränder der Einführungsschlitze 21, 22 sind zugespitzt abgeflacht, wie insbesondere in Figuren 5 und 6 erkennbar ist und die Vorsprünge 16, 17 sind sowohl an ihrer Außenfläche als auch an ihren beiden schmalen Enden so abgerundet, daß sie in Umfangsrichtung kantenfreie Nockenform erhalten (Figur 5).

Zur Einrichtung der Punktionskanüle als verkaufsfertiges Produkt werden das Kunststoffkapillar 11 und die Stahlkanüle 13 zusammengesteckt, bis die hinteren Flanken der beiden radialen Vorsprünge 16, 17 des Katheteransatzes 10 gegen den Grund 21a, 22a der Einführungsschlitze 21, 22 des Ringbundteiles 20 anstoßen (Figur 2). In diesem Zustand ist die Griffplatte 14 des Katheteransatzes 10 um 90° zu der Griffplatte 15 des Kanülenansatzes 12 versetzt. Die Griffplatte 14 wird sodann in Richtung des Pfeiles A in Figur 2 gedreht, während der Kanülenansatz 12 an der Griffplatte 15 festgehalten wird. Dabei unterfahren die nockenartigen Vorsprünge 16 und 17 die Schalen 23, 24 des Ringbundteiles 20 und drücken ihre den Einführungsschlitzen 21, 22 zugewandten Stegpartien um die Höhendifferenz zwischen dem Vorsprung-Außenradius und dem Ringbund-Innenradius elastisch nach außen, bis sie in die Lochöffnungen 30, 31 eintreten. Da die Stegpartien in diesem Moment radial zurückfedern, rasten die Vorsprünge 16, 17 in die Lochöffnungen 30, 31 ein und stellen eine sichere formschlüssige Verbindung zwischen Kanülenansatz 12 und Katheteransatz 10 her. Die Verriegelung ist bei normaler Handhabung sowohl in axialer als auch in Umfangsrichtung wirksam. Infolge der Nockenform der Vorsprünge 16, 17 und der federnden Auslenkbarkeit des Ringbundteiles ist jedoch durch Drehung der Ansätze 10, 12 relativ zueinander eine Dekonnektion möglich, wenn nach erfolgreicher Punktion die Stahlkanüle 13 aus dem Kunststoffkapillar 11 herausgezogen werden soll. Wenn das Kunststoffkapillar 11 als Venenverweilkatheter in dem Blutgefäß liegenbleibt, dienen die radialen Vorsprünge 16 und 17 in üblicher Weise der Verbindung mit einem Anschlußstück eines Infusionssystems oder dergleichen.

**Patentansprüche**

1. Punktionskanüle, bestehend aus einem Kunststoffkapillar (11), das an seinem patientenfernen Ende einen hohlen Katheteransatz (10) aufweist und aus einer in das Kunststoffkapillar (11) einschiebbaren Stahlkanüle (13) mit einem Kanülenansatz (12), der mit dem Katheteransatz (10) zusammensteckbar und in zusammengestecktem Zustand durch eine Verriegelungsvorrichtung sicherbar ist, die mindestens einen äußeren radialen Vorsprung (16, 17) an dem Katheteransatz (10) sowie mindestens einen Einführungsschlitz (21, 22) und eine Ausnehmung zur Aufnahme des Vorsprunges (16, 17) in einem Ringbundteil (20) des Kanülenansatzes (12) aufweist,
**dadurch gekennzeichnet,**
daß die Ausnehmung als geschlossene Lochöffnung (30, 31) ausgebildet ist, in die der auf der Außenfläche und an seinen beiden Enden nockenartig abgerundete radiale Vorsprung (16, 17) durch Relativdrehung von Katheteransatz (10) und Kanülenansatz (12) einrastend einführbar ist, und daß der der Lochöffnung (30, 31) zugewandte Seitenrand des Einführungsschlitzes (21, 22) lippenartig abgeflacht ist.

2. Punktionskanüle nach Anspruch 1,
**dadurch gekennzeichnet,**
daß der axiale Ringbundteil (20) elastisch radial auslenkbar ist.

3. Punktionskanüle nach Anspruch 1 oder 2,
**dadurch gekennzeichnet,**
daß der Grund (21a, 22a) des Einführungsschlitzes (21, 22) auf gleicher Umfangslinie mit dem patientenfernen Rand der Lochöffnung (30, 31) liegt.

4. Punktionskanüle nach einem der Ansprüche 1 bis 3,
**dadurch gekennzeichnet,**
daß der Vorsprung (16, 17) in Draufsicht keilförmig gestaltet ist und daß die Form der Lochöffnung (30, 31) der Form des Vorsprunges (16, 17) im wesentlichen angepaßt ist.

**Claims**

1. A puncture cannula comprising a plastics capillary (11) with a hollow catheter hub (10) at its distal end, and a steel cannula (13) to be pushed into said plastics capillary (11), with a cannula hub (12) that may be assembled with said catheter hub (10) and may be secured in said assembled state by means of a locking means that has at least one outer radial projection (16, 17) at said catheter hub (10), as well as at least one insertion slit (21, 22) and a recess for receiving said projection (16, 17) in an annular collar portion (20) of said cannula hub (12),

characterised in

that said recess is in the shape of a closed hole opening (30, 31) into which said radial projection (16, 17), rounded cam-like on the outer surface and the two ends, may be inserted for engagement by a relative rotation of said catheter hub (10) and said cannula hub (12) and that the lateral edge of said insertion slit (21, 22) facing said hole opening (30, 31) is flattened lip-like.

2. The puncture cannula of claim 1, characterised in that said axial annular collar portion (20) is resiliently deflectable in the radial direction.

3. The puncture cannula of claim 1 or 2, characterised in that the bottom (21a, 22a) of said insertion slit (21, 22) lies on the same circumferential line as the distal edge of said hole opening (30, 31).

4. The puncture cannula of one of claims 1 to 3, characterised in that said projection (16, 17) is wedge-shaped in top plan view and that the shape of said hole opening (30, 31) is substantially adapted to the shape of said projection (16, 17).

**Revendications**

1. Canule pour ponction constitué par un capillaire en matière synthétique (11), qui présente , à son extrémité éloignée du patient, un embout creux de cathéter (10), et par une canule en acier (13) qui peut être insérée dans le capillaire en matière synthétique (11) et comporte un embout de canule (12), qui peut être réuni par enfichage à l'embout (10) du cathéter et peut être bloqué à l'état réuni par enfichage, par un dispositif de verrouillage, qui présente au moins une partie saillante radiale extérieure (16,18) située sur l'embout (10) du cathéter ainsi qu'au moins une fente d'introduction (21,22) et un évidement servant à recevoir la partie saillante (16,17) dans une partie formant collet annulaire (20) de l'embout (12) de la canule,
caractérisée par le fait que l'évidement est réalisé sous la forme d'une ouverture en forme de trou (30,31) fermée, dans laquelle la partie saillante radiale (16,17), qui est arrondie avec une forme de came sur la surface extérieure et à ses deux extrémités, peut être insérée avec encliquetage moyennant une rotation relative de l'embout (10) du cathéter et l'embout (12) de la canule, et que le bord latéral, tourné vers l'ouverture (30,31) en forme de trou, de la fente d'introduction (21,22) est aplati avec une forme de lèvre.

2. Canule pour ponction suivant la revendication 1, caractérisée en ce que la partie formant collet annulaire axial (20) peut fléchir élastiquement radialement.

3. Canule pour ponction selon la revendication 1 ou 2, caractérisée en ce que le fond (21a,22a) de la fente d'insertion (21,22) est situé sur la même ligne de contour que le bord, éloigné du patient, de l'ouverture (30,31) en forme trou.

4. Canule pour ponction selon l'une des revendications 1 à 3, caractérisée en ce que la partie saillante (16,17) est agencée avec une forme en coin selon une vue en plan et que la forme de l'ouverture (30,31) en forme de trou est essentiellement adaptée à la forme de la partie saillante (16,17).

FIG. 1

FIG. 2

FIG. 3

FIG. 4

FIG. 5

FIG. 6